# EUROPÄISCHE PATENTSCHRIFT

(11) **EP 0 794 770 B1**
(45) Veröffentlichungstag und Bekanntmachung des Hinweises auf die Patenterteilung: **07.03.2001**
(21) Anmeldenummer: 95941659.5
(22) Anmeldetag: 04.12.1995
(51) Int. Cl.: A61K 9/70, A61K 31/445

(54) **TRANSDERMALE DARREICHUNGSFORM MIT ANTIHISTAMINISCH WIRKSAMEN LORATIDINMETABOLIT**
TRANSDERMAL PREPARATION CONTAINING A LORATIDINE METABOLITE WITH ANTIHISTAMINIC ACTIVITY
PREPARATION TRANSDERMIQUE CONTENANT UN METABOLITE DE LORATIDINE A ACTION ANTIHISTAMINIQUE

(30) Priorität: 02.12.1994 DE 4442999
(43) Veröffentlichungstag der Anmeldung: 17.09.1997
(73) Patentinhaber: HEXAL AG, D-83607 Holzkirchen (DE)
(72) Erfinder: KLOKKERS, Karin, D-83607 Holzkirchen (DE); FISCHER, Wilfried, D-83607 Holzkirchen (DE)
(74) Vertreter: Boeters, Hans Dietrich, Dr.
(86) Internationale Anmeldenummer: EP9504761
(87) Internationale Veröffentlichungsnummer: WO9616641

(56) Entgegenhaltungen:
- US-A- 4 910 205
- HOSP. FORMUL., Bd. 28, Nr. 2, 1993 Seiten 137-153, XP 000566896 R.A. QUERCIA ET AL. 'Focus on loratidine: a new second-generation nonsedating H1-receptor antagonist.'
- PHARMAZIE, Bd. 49, Nr. 10, 1994 Seiten 736-739, XP 000566477 D. ZHONG ET AL. 'HPLC-determination of loratidine and its active metabolite descarboethoxyloratidine in human plasma.'

## Beschreibung

Die Erfindung betrifft eine pharmazeutische Zusammensetzung zur systemischen transdermalen Verabreichung mit einem Gehalt an einem aktiven Loratidin-Metaboliten als Wirkstoff.

Loartidin (Ethyl-[4- (8-chlor-6,11-dihydro-5H-benzo-[5,6]-cyclo-hepta-[1,2-b]-pyridin-11-yliden)-1-piperidincarboxylat]) ist ein Antihistaminikum, das als Saft oder in Form von Tabletten in den Handel kommt. Der Wirkstoff wird im Körper metabolisiert.

Es besteht nun ein Bedürfnis, den antihistaminischen Effekt zu verbessern und eine systematische Applikation vorzusehen. Bei Untersuchungen, die dafür vorgenommen wurden, wurde nun überraschenderweise festgestellt, daß ein aktiver Loratidin-Metabolit eine ausreichende Stabilität besitzt, um als Wirkstoff in einer pharmazeutischen Zusammensetzung zur systematischen transdermalen Verabreichung vorgesehen zu werden.

Für relevanten Stand der Technik sei beispielsweise verwiesen auf DE-A-3 212 053, GB-A-2 098 865, Remington's Pharmaceutical Sciences, Auflage 16, Mack-Verlag und Sucker, Fuchs & Spieser, Pharmazeutische Technologie, Auflage 1, Springer-Verlag. Außerdem ist aus US-A-4,910,205 eine pharmazeutische Zusammensetzung mit Pflasterstruktur mit Reservoir zur systemischen transdermalen Verabreichung bekannt, enthaltend einen aktiven Loratidin-Metaboliten als Antihistaminikum, wobei der Wirkstoff in dem Reservoir in einem Gemisch aus pharmazeutisch akzeptablen flüchtigen Lösungsmitteln, wie Methanol, Isopropanol oder Ethanol, einem Festsäureester und einem ätherischen Öl vorliegt. Beispielhaft wird ein Flux von 2,26 mg Wirkstoff/15 cm² Humanhaut/24 h angegeben. Es bestand jedoch noch ein Bedürfnis, diesen Stand der Technik zu verbessern.

Die der Erfindung zugrundeliegende Aufgabe wird nun durch eine pharmazeutische Zusammensetzung mit transdermaler Pflasterstruktur zur systematischen Verabreichung gelöst, die einen aktiven Loratidin-Metaboliten als Antihistaminikum enthält, wobei diese Zusammensetzung dadurch gekennzeichnet ist, daß der Wirkstoff in einer Matrix auf Acrylatbasis vorliegt.

Ein Loratidin-Metabolit kann von den Irotec Laboratories (Country Cork, Irland) bezogen werden und besitzt die folgende Formel:

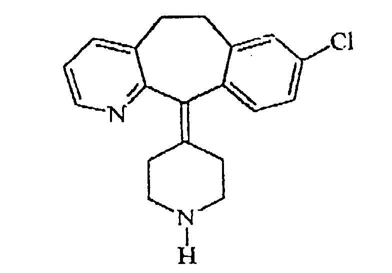

Erfindungsgemäß kann die Pflasterstruktur durch eine Matrix auf Acrylatbasis vorgesehen werden, die in üblicher Weise auf einer wasserundurchlässigen Trägerschicht ausgebildet ist, wobei zusätzlich eine entfernbare, die Matrix schützende Deckschicht vorgesehen sein kann.

Bei dem Material der Matrix kann es sich erfindungsgemäß um ein nicht-quellbares Acrylatpolymeres handeln, beispielsweise um Durotack 280-2416 (Delft National Chemie, Zotphen, Niederlande). Für die Matrix kann ferner auf ein Polymeres gemäß EP-A-0 155 229 verwiesen werden.

Nachstehend wird die Erfindung beispielhaft näher erläutert.

### Test in vitro

Es wurde ein Diffusionstest in vitro gemäß GB-A-2 098 865 durchgeführt. Dabei wurde der aktive Loratidin-Metabolit auf eine Seite eines isolierten intakten Maushautsegments einer Fläche von 2,5 cm² aufgebracht. Die andere Seite des Hautsegements stand mit einer 0,9-proz. Natriumchloridlösung mit einem zusätzlichen Gehalt an 0,05 % Natriumazid in Kontakt. Die Menge an Wirkstoff der in die Salzlösung eintrat, wurde in üblicher Weise durch HPLC (HP-Flüssigchromatographie) verfolgt. Einzelheiten sind im folgenden zusammengestellt.

| | |
|---|---|
| Aktiver Loratidin-Metabolit | 16,6 mg/ml |
| Propylenglykol : Wasser (1:1) | 5,0 ml |

| Penetrationsraten pro 2,5 cm² | Zeit [h] | Menge [µg/cm²] | Flux [µg/cm²/24h] |
|---|---|---|---|
| | 3 | 9,0 | 72 |
| | 6 | 85,0 | 341 |
| | 9 | 175,2 | 467 |
| | 14 | 333,6 | 572 |
| | 19 | 508,3 | 642 |
| | 24 | 578,8 | 579 |
| | 32 | 884,2 | 663 |

### Beispiel 1

| | |
|---|---|
| Loratidin-Metabolit | 2,0 g |
| Duro-Tak 1753 | 98,0 g |

Die vorstehend angegebenen Rohstoffe werden bis zur klaren Lösung gemischt. Die Lösung wird auf eine silikonisierte Folie oder Papier gestrichen, so daß ein Flächengehalt von 100 g/m² resultiert. Auf die getrocknete Matrix wird eine transparente Polypropylen- oder Polyesterfolie laminiert. Aus dem Laminat werden die fertigen Pflaster mit Größen zwischen 10 cm² (entsprechend 2 mg Wirkstoff) oder 40 cm² (entsprechend 8 mg Wirkstoff) gestanzt.

## Patentansprüche

1. Pharmazeutische Zusammensetzung mit transdermaler Pflasterstruktur zur systemischen transdermalen Verabreichung, enthaltend einen aktiven Loratidin-Metaboliten als Antihistaminikum, dadurch **gekennzeichnet**, daß der Wirkstoff in einer Matrix auf Acrylatbasis vorliegt.

2. Pharmazeutische Zusammensetzung nach Anspruch 1, gekennzeichnet durch ein nicht-quellbares Acrylatpolymeres als Matrix.

## Claims

1. Pharmaceutical composition having a transdermal patch structure for systemic transdermal administration, comprising an active loratidine metabolite as antihistamine, characterised in that the active ingredient is present in an acrylate-based matrix.

2. Pharmaceutical composition according to claim 1, characterised by a non-swellable acrylate polymer as matrix.

## Revendications

1. Composition pharmaceutique ayant une structure de timbre transdermique pour administration transdermique systémique, contenant un métabolite de loratidine actif en tant qu'antihistaminique, caractérisée en ce que la substance active est présente dans une matrice à base d'acrylate.

2. Composition pharmaceutique selon la revendication 1, caractérisée par un polymère d'acrylate non gonflable comme matrice.
